# EUROPEAN PATENT SPECIFICATION

(11) **EP 1 848 346 B1**
(45) Date of publication and mention of the grant of the patent: **19.10.2011**
(21) Application number: 06719810.1
(22) Date of filing: 27.01.2006
(51) Int. Cl.: A61B 17/072

(54) **Surgical fastener buttress materials**
Versteifungsmaterial für chirurgische Befestigungsvorrichtungen
Materiaux de renfort pour appareils chirurgicaux de fixation

(30) Priority: 31.01.2005 US 47477
(43) Date of publication of application: 31.10.2007
(73) Proprietor: Synovis Life Technologies, Inc., St. Paul, MN 55114-1024 (US)
(72) Inventor: ORAY, B., Nicholas, Woodbury, MI 55125 (US); MOORADIAN, Daniel, L., Eagan, MI 55123 (US)
(74) Representative: Molnia, David
(86) International application number: PCT/US2006/003121
(87) International publication number: WO 2006/083748

(56) References cited:
- US-A- 5 752 965
- US-A1- 2002 165 559
- US-A1- 2003 120 284
- US-A1- 2004 093 029
- US-B1- 6 379 702

## Description

### FIELD OF THE INVENTION

This invention relates to surgical fastener, and to buttress materials adapted to be used in combination with such surgical fasteners.

### BACKGROUND OF THE INVENTION

Surgical fasteners are commonly used in surgeries to perform a variety of functions, for example to remove parts of organs (e.g., to resect tissue), to cut through organs and tissues (transection), and to create connections between structures (to create anastomes). A variety of surgical fasteners are currently in use and include clip appliers, surgical staplers and other devices.

Surgical staplers are commonly used in a variety of surgical procedures including vascular, bariatric, urologic, gynecologic, thoracic and pediatric surgeries. For example, one such thoracic procedure is lung volume reduction, which resects a portion of lung to treat emphysema or other conditions. Also, one such bariatric procedure is the gastric bypass procedure, which resects a portion of the stomach to create a smaller stomach to aid in weight loss.

Surgical staplers are also provided in many forms, including linear staplers, circular staplers, and contoured or curved staplers. Surgical staplers commonly include a pair of apposed working surfaces. In the case of a linear stapler, two apposing jaws are provided, one holding a cartridge of staplers and the other being an anvil member against which the staples are to be fired. When the stapler is fired, it simultaneously emits a plurality of rows of closely spaced staples from the cartridge, and through the patient's own tissue.

In some instances, air and/or fluid leaks and/or excessive bleeding in the staple line have been reported. The term "staple line" is used herein to refer to any row of stapled tissue, and the staple line can be linear, circular or curved, depending on the type of stapler used. Leaks can occur in the cut line, and/or in the staple holes themselves. Frequently in the case of thoracic procedures, the diseased lung tissue is thin and friable and can tear at the staples as the lungs re-inflate. These air leaks can be persistent and can extend the hospital stay for a patient by weeks. As a means to alleviate these leakage problems, surgeons often reinforce the staple line by applying a buttress or pledget material to the desired stapling site, and stapling through the buttress material. The buttress material provides reinforcement to the friable tissue as it reduces the changes of tissue tearing at the staple line, and reduces staple pullout in friable tissue. The buttress material also creates a stronger staple line for increase security against air and/or fluid leaks and/or excessive bleeding both during surgery and in the crucial weeks that follow.

A variety of materials have been described for use as staple line buttress materials, including those formed of specially treated tissue (e.g., bovine pericardial tissue), alginate, polyglycolic felt, and Teflon brand materials, however only a handful have been found suitable for commercial use. These buttress materials are typically mounted in a releasable manner onto the working surfaces of a surgical stapler such that upon firing, the patient's tissue is sandwiched between the strips of buttress material.

Buttress materials are typically releasably mounted upon the working surfaces of a surgical stapler by either mechanical and/or chemical means. In some instances, an adhesive is applied to one or both surfaces of the stapler itself, or to a surface of a buttress material strip, and the strip is then applied to the corresponding working surface. For example, in the case of linear staplers, the buttress material is often attached with an adhesive to each apposing jaw. Current adhesives are typically biocompatible gels that are applied to the buttress material itself in order to hydrate the material and to create a temporary bond between the material and the stapler.

In many cases, it is generally necessary to properly align and position the buttress material strip onto the working surfaces of the stapler. An improperly aligned buttress material often results in a failed staple line. Apparatuses that include an alignment feature have been developed to aid a user in properly aligning a buttress material onto working surfaces of a stapler. For example, such an apparatus is described in Applicant's U.S. Patent No. 5,752,965. The preamble of claim 1 is based on this document.

### SUMMARY OF THE INVENTION

The invention provides an improved buttress material. Current methods of positioning the buttress material onto a working surface of a stapler typically involve at least two steps, a step of applying an adhesive material to the buttress material (or to the working surface of the surgical stapler) and then applying the buttress material to the working surface. The invention makes possible a one-step method of applying a buttress material to a working surface of a stapler. Also, current methods of aligning the buttress material onto a working surface of a stapler are not fail proof, even with the use of aligning devices. Often times, when a user applies an adhesive to either the buttress material or to the working surface of the stapler, too much adhesive is applied. This excess adhesive often causes the working surface to become slippery and the buttress material itself slides along the surface and becomes misaligned. Thus, the invention also provides a method of applying a buttress material to a working surface of a stapler that substantially reduces misalignments resulting from the user misapplication of the adhesive.

### BRIEF DESCRIPTION OF THE DRAWINGS

Figure 1 is a perspective view showing an article of buttress material according to an embodiment of the invention.
Figure 2 is a perspective view showing the method of engagement between a surgical stapler and two articles of buttress material according to an embodiment of the invention.
Figure 3 a is a partially exploded view of an aligning apparatus adapted to incorporate two articles of buttress material according to an embodiment of the invention.
Figure 3b is a cross sectional view of the apparatus in Figure 3a taken along line 3B-3B.
Figure 4a is a perspective view illustrating the method of engagement between a surgical stapler and an aligning apparatus which incorporates two articles of buttress material according to an embodiment of the invention.
Figure 4b is a cross sectional view of the apparatus in Figure 4a taken along line 4B-4B.

### DETAILED DESCRIPTION OF THE PREFERRED EMBODIMENTS

In some embodiments, the invention provides a suture line buttress material having at least one adhesive surface, the material being packaged and provided in a manner that permits the surface to be aligned with and releasably cover the working surface of a surgical fastener. The term "suture line" is used herein to refer to any row of sutured tissue. The suture line can be sutured using staples, clips and the like. Also, the suture line can be linear, circular, curved or of any other suitable pattern or design depending on the type of surgical fastener used. The buttress material is adapted for use with any surgical fastener known in the art. In preferred embodiments, the surgical fastener preferably comprises a surgical stapler, and more preferably comprises a linear stapler.

Buttress materials are often provided in the form of a strip or other configuration where there are two major surfaces, one surface adapted to come into contact with a working surface of a surgical fastener and another surface adapted to be positioned against body tissue. The adhesive surface of the buttress material is provided on a surface that will come into contact with the working surface of the surgical fastener. The adhesive is also sufficiently tacky to permit the material, preferably without extraneous means such as removable sleeves, strings or added gels, to be retained upon the working surface of the surgical fastener in the course of the surgical fastener being positioned within the surgical site, and then to be removed from the working surface upon application of the surgical fastener.

Buttress materials having at least one adhesive surface provide several advantages, including the ability to avoid the need for a separate step for applying an adhesive gel. This improves the ease-of-use of loading the buttress material within the surgical fastener because currently used two-step loading processes are now converted to a one-step loading process. Furthermore, this elimination of the second step helps to reduce user misapplication of the adhesive.

The buttress material having at least one adhesive surface can be comprised of a single material or a composite of two or more of the same or different materials. In preferred embodiments, the buttress material includes at least one material having sufficient mechanical strength to reinforce a suture line (hereinafter "reinforcing material"). The composite of materials can be provided as a laminate of materials, a homogenous mixture of materials, a graded mixture of materials or as other suitable formations.

The reinforcing material can also be formed from synthetic or natural tissue. In certain embodiments, the reinforcing material is formed from natural animal tissue. In some cases, the natural animal tissue is a fibro-serous or serous membrane. In preferred cases, the natural animal tissue is a fibro-serous membrane comprising pericardium, preferably bovine pericardium. In some embodiments, the natural animal tissue can be a tissue of the type described in any of Applicant's own U.S. Patent Nos. 5,503,638, 5,575,803,5,549,628,6,468,313,6,312,474 and 6,652,594.

In particularly preferred cases, the reinforcing material comprises treated bovine pericardium available from the applicant's assignee and sold under its trademark Peri-Strips Dry^{®}. The use of this particular type of material is advantageous in that it has a much higher density of collagen than most other connective tissues and is cross linked with glutaraldehyde. The high density of the bovine pericardium provides increased structural integrity about the suture line and he cross linking with glutaraldehyde is advantageous in that it decreases the antigenicity of the tissue, thereby resulting in little or no inflammatory reaction with the adjoining body tissue. In another preferred embodiment of the invention, the reinforcing material comprises treated bovine pericardium available from the applicant's assignee and sold under its trademark Veritas^{®}. While the preferred embodiment of the present invention employs bovine pericardium as the buttressing biomaterial, it is to be readily understood that other suitable pericardium or dura mater may be employed, including but not limited to equine, porcupine, ovine, and human, as well as bio-compatible synthetic materials.

In a preferred embodiment of the present invention, the reinforcing material comprises a smooth, dense, nonporous membrane. A tissue of cross-linked bovine pericardium, e.g., Peri-Strips Dry^{®} material is an example of a such a dense membrane. In other embodiments, the reinforcing material resists or substantially resists degradation *in vivo.* For example, such a reinforcing material preferably undergoes little or no hydrolysis once in contact with bodily fluid or,saline solution. Cross-linked bovine pericardium, e.g., Peri-Strips Dry^{®} material is also an example of a material which resists degradation *in vivo.* In other cases, a remodelable material is provided which is integrated into the surrounding body tissue over time. Preferably, the remodelable material comprises non-crosslinked animal tissue, for example bovine pericardium. Veritas^{®} material is an example of a such a remodelable material.

In additional embodiments, the reinforcing material exhibits superior tensile strength, leak resistance and burst resistance. For example, the material preferably has a pre-implant tensile strength wherein the peak load is at least about 2000 grams, more preferably at least about 3000 grams and optimally at least about 4000 grams. In other cases, the material has a pre-implant tensile strength which does not significantly decrease over time once implanted *in vivo.* The pre-implant tensile strength of a material can be measured using an MTS Q45 uniaxial tensile tester. The reinforcing material also preferable has a leak pressure of at least about 90 mm Hg, more preferably at least about 150 mm Hg. In other embodiments, the reinforcing material has a burst pressure of at least about 90 mm Hg, more preferably at least about 150 mm Hg and optimally at least about 200 mm Hg. The leak pressure and burst pressure can be measured by introducing blue dye onto one side of the suture line at a constant rate and then introducing pressure. The leak pressure is the pressure at which leaking of the blue dye through the suture line is observed. The burst pressure is recorded when the pressure reaches a plateau or drops abruptly as the suture line bursts. Cross-linked bovine pericardium, e.g., Peri-Strips Dry^{®} material is also an example of a material exhibiting superior tensile strength, leak resistance and burst resistance.

The buttress material can be provided with an adhesive surface in a variety of ways. In some embodiments, the buttress material includes a single material having adhesive properties in its own right. The single material also preferably has sufficient mechanical strength to reinforce a suture line. For example, a hydrogel film can be provided as the single material, wherein the hydrogel is itself provided with sufficient mechanical strength. In this case, any surface of the buttress material could be provided as the adhesive surface to be contacted with a working surface of a surgical fastener.

In other embodiments, an adhesive material is pre-applied upon a surface of the buttress material (which can include a single material or composite of one or more materials). In these cases, the one or more materials comprising buttress material generally have reinforcing and buttressing properties rather than adhesive properties, since a separate adhesive is pre-applied to a surface. However, this is by no means required and the one or more materials can certainly include a material having adhesive properties. In this case, the surface of the buttress containing the pre-applied adhesive is provided as the adhesive surface. The adhesive material is preferably formed upon a surface via a layer of film, although other configurations are also possible.

The adhesive can be pre-applied upon one or more surfaces of the buttress material according to any known methods in the art using conventional coating equipment and materials.
Suitable coating methods include, for instance, air knife, brush, calendar, casting, curtain, dip, extrusion, blade, knife, gravure, kiss roll, knife over blanket, knife over roll, offset gravure, reverse roll, reverse smoothing roll, rod, sprays, squeeze roll, *in vivo* polymerization, and powdered resin coating. In turn, the coated material can be in any suitable form, e.g., the form of a powdered resin composition, aqueous or solvent based lattice, emulsion or dispersion.

The adhesive can be applied to one surface, two or more surfaces, or to all surfaces of the buttress material. In cases where the buttress material is porous or otherwise capable of being saturated, the adhesive can be applied to saturate the entire buttress. Preferably the hydrogel is applied to only one side, with the opposite side being left uncoated, or itself treated or provided with a suitable coating or properties. The adhesive is preferably applied to one or more surfaces as a thin layer, e.g., between about 0.1 mm and about 1.0 mm in nominal thickness, and more preferably between about 0.25 mm and about 0.75 mm nominal thickness.

A variety of materials can be provided as the adhesive material. In preferred embodiments, the adhesive material comprises a biocompatible gel. In particularly preferred embodiments, the adhesive material comprises a hydrogel, which can also be described as a polymeric water-containing gel. Suitable hydrogels include those based on natural hydrogels (e.g., artificial silk, cellulosic materials) and synthetic hydrogels, such as methacrylic and acrylic esters, acrylamide hydrogels, hydrogels based on N-vinyl-2-pyrrolidone, and polyelectrolyte complexes and charged hydrogels, including blends, mixtures and copolymers thereof. The inventors have discovered that suitable hydrogels provide an optimal combination of such properties as adhesion to the buttress material of choice, adhesion to the working surfaces of a surgical fastener, flexibility, contour formation, biocompatibility, biostability, and the ability to be sterilized.

Examples of suitable hydrogels include those available under the tradename Aquatrix II™ from Hydromer, Inc., a corporation located at Branchburg, New Jersey. In a particularly preferred embodiment, the hydrogel comprises a poly(N-vinyl lactam)-chitosan gel. Such gels ar described in U.S. Patent Nos. 6,379,702, 6,365,664, 6,121,375, 5,420,197, 5,258,421, and 5,156,601. Chitosan is a deacetylated chitin, and is a linear polysaccharide of deacetylated N-acetyl-D-glucosamine. Poly(N-vinyl lactam) s such as polyvinylpyrrolidone (PVP) have been used in pharmaceuticals, in certain types of films, and in some cosmetic products. They can be used to provide dermatologically-compatible gels having a hydrophilic absorbent property and K values of less than 60. Such.materials are preferably stable, hydrophilic gels which comprise a blend of acid-neutralized chitosan and a poly(N-vinyl lactam), with or without a plasticizer, the poly(N-vinyl lactam) having a K value of less than about 60 and mole equivalents of acid groups of at least about 1.4.

Such preferred gels can be prepared by mixing aqueous poly(N-vinyl lactam) solution and acid neutralized chitosan in aqueous solution at a poly(N-vinyl lactam)/chitosan weight ratio of from about 12/1 to about 1/1, preferably from about 10/1 to about 5/1, to form a blend at about 5 wt. % to 40 wt. % total polymer concentration, preferably from about 12.5 wt. % to about 25 wt. % polymer concentration, and allowing the blend to cure until a gel is formed.

In some embodiments, the adhesive surface has a predetermined adhesive strength or tack, the predetermined adhesives strength being reduced when the adhesive surface is hydrated. The terms "tack" and "adhesive strength" are used herein to describe the stickiness of the adhesive. The tack of an adhesive is typically the pull resistance (measured in dynes) exerted by the adhesive completely adhering to two surfaces being pulled apart. The higher the pull resistance, the higher the tack of the adhesive. Thus, when the adhesive material is outside of the body and in a dry form, it has sufficient tack to prevent the buttress material from being removed or realigned along the working surfaces of the surgical fastener. However, when the buttress material is placed *in vivo* where it comes into contact with tissue and body fluids, it is hydrated and this causes the surface to be easily removed from the working surfaces of the surgical fastener. Therefore, in preferred cases, the adhesive surface is packaged and provided in a manner that has sufficient tack to be securely applied to a working surface of a surgical fastener, this tack being reduced when the adhesive surface is positioned within the surgical site.

In preferred embodiments, the adhesive material of this invention can be used to deliver one or more bioactive agents, including both locally at the site of placement, or more broadly, including systemically through the patient. Such bioactive agents can serve to improve the function of the adhesive itself and/or they can serve related or unrelated functions within the body. In one embodiment, for instance, the adhesive can itself be used largely as a bioactive delivery device, with its function as a buttress material being ancillary to that. In preferred cases, one or more bioactive agents are provided that would improve healing of the stapled or otherwise sutured tissues. Examples of suitable healing bioactive agents include, but are not limited to antibiotics, anti-inflammatory agents, growth factors, and cytokines. Coagulants can also be provided to reduce the bleeding and radioactive sees can be provided to monitor the location of the buttress material within the body.

In some embodiments, the invention also provides surgical combinations, which include a buttress material according to any of the embodiments already described. For example, in some cases, a combination including a surgical fastener and at least one article of buttress material is provided. Any surgical fastener known in the art can be used with this combination. In preferred embodiments, the surgical fastener preferably comprises a surgical stapler, and more preferably comprises a linear stapler. In other cases, a combination including an apparatus for equipping a surgical fastener with a buttress material and at least one article of buttress material is provided. Suitable apparatuses for equipping a surgical fastener with a buttress material are described in U.S. Patent Nos. 5,752,965 and 6,656,193 and in U.S. Publication Nos. 20040093029, 20030120284 and 20020165563. Such apparatuses are useful for disposing one or more articles of buttress material in releasable attachment with working surfaces of a surgical fastener such that the articles are automatically lined up with those surfaces. The at least one article of buttress material can be provided as installed within a buttress material or as a separate component that is part of a surgical kit in which the buttress material is inserted within the apparatus at the surgical site.

Exemplary surgical methods of using the buttress material are also disclosed. Buttress materials of the invention can be used in a variety of surgeries, including vascular, bariatric, urologic, gynecologic, thoracic and pediatric surgeries. Common bariatric surgeries include gastric bypass and gastric bending. Gastric bypass surgeries also include the following types of surgeries: roux-en-y gastric bypass (RYGB), vertical banded gastroplasty, and biliopancreatic diversion. Common thoracic surgeries include blebectomies, bullectomies, wedge resections, segmentectomies, lobectomies, pneumonectomy, lung volume reduction surgery, and other resections of the lung and bronchus.

An exemplary surgical method may generally include providing one or more articles of buttress material to a working surface of a surgical fastener, positioning the surgical fastener about a section of body tissue, and applying the fastener to form a reinforced suture line. In cases where the adhesive material loses adhesive strength when hydrated *in vivo,* it is desirable to position the surgical fastener about a section of body tissue for a period of time long enough to hydrate the adhesive material so that the articles of buttress material will be easily released from the surgical fastener working surfaces.

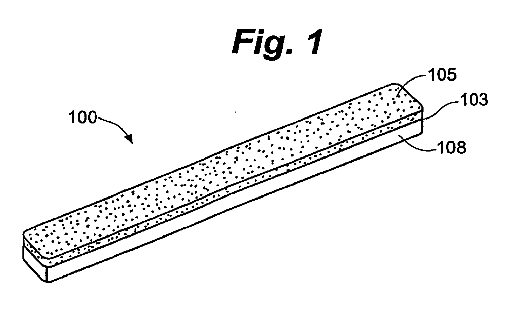

The use of a buttress material of this invention will now be described. Figure 1 illustrates an article of buttress material 100 having a layer of adhesive material 105 pre-applied upon a surface 103 of the reinforcing material 108. The adhesive material 105 comprises any suitable adhesive material and the reinforcing material 108 comprises any suitable reinforcing material. The buttress material 100 is sized and configured to be positioned and aligned upon a linear stapler jaw, with the surface 103 containing the adhesive 105 being in direct contact with the jaw surface.

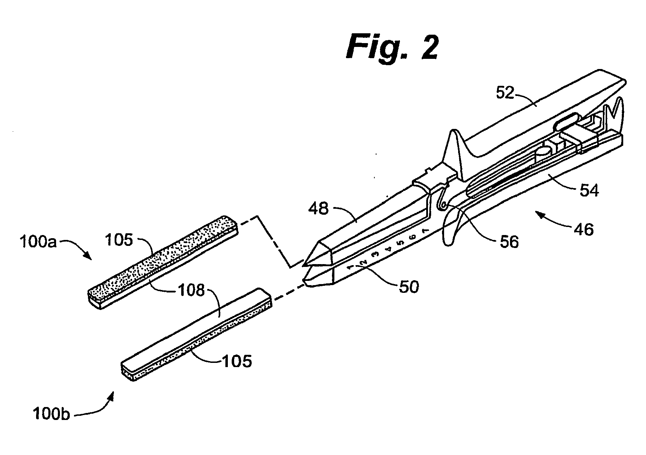

Figure 2 illustrates a generic linear surgical stapler 46 having a staple cartridge supporting jaw 48 and an anvil supporting jaw 50 extending from a two-part handle, which in turn includes a movable part 52 and a stationary part 54 which is pivotally and removably hinged at 56 to the movable part. The cartridge supporting jaw 48 holds a cartridge body (not shown) containing a plurality of staples disposed in rows oriented longitudinally to the jaw 48 in opposition to an anvil (not shown) supported by jaw 50 when the members 48 and 50 are in their closed position. Two articles of buttress material 100a and 100b are provided sized and shaped for mounting onto the working surfaces of the parts 52 and 54 of the surgical device 46. As illustrated, article 100a is positioned so that its layer of pre-applied adhesive material 105 is oriented upwardly so as to adhere to the working surface of the cartridge supporting jaw 48. Likewise, article 100b is positioned so that its layer of pre-applied adhesive material 105 is oriented downwardly so as to adhere to the working surface of the anvil supporting jaw 50.

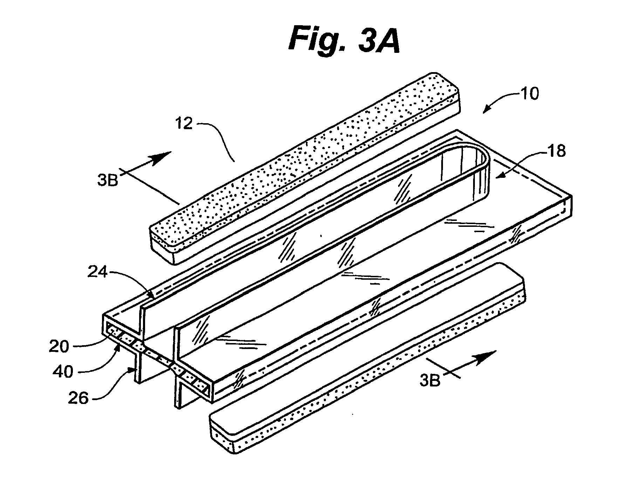

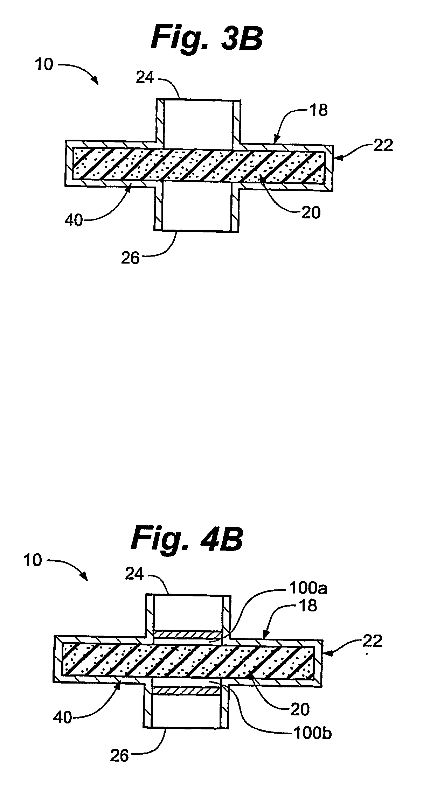

Figures 3a-3b show an apparatus 10 for equipping a surgical fastener with a buttress material. The apparatus is adapted to incorporate articles of buttress material 100a and 100b in accordance with a particularly preferred embodiment. In this embodiment, the apparatus 10 is configured for use with a linear stapler.

Referring collectively to Figures 3a-3b, the apparatus 10 includes an alignment frame 18 and an internally disposed pressure equalization member 20. The alignment frame 18 can be configured in any way so as to retain articles 100a and 100b of buttress material in releasable attachment within the aligning frame 18 such that the articles 100a and 100b will be automatically lined up with the apposed jaw members of the linear stapler. In the illustrated embodiment, the aligning frame 18 includes a generally planar sheath portion 22, a first guide channel 24 and a second guide channel 26. Sheath portion 22 includes an internally disposed receiving area 40. The pressure equalization member 20 is internally disposed within the receiving area 40 and is preferably constructed from a deformable yet resilient substrate, such as foam plastic, rubber, and/or any number of suitable materials having similar properties. By providing such a uniform distribution of pressure, the pressure equalization member 20 ensures articles 100a and 100b will more readily conform to the shape and contour of the apposed working surfaces during the step of closing the surgical stapler about the apparatus 10. The resiliency of the pressure equalization member 20 is also advantageous in that it serves to releasably bias articles 1 00a and 100b within the receiving area 40 of the alignment frame 18.

The first guide channel 24 and the second guide channel 26 are dimensioned to regulate the engagement of the apposed working surfaces of a surgical stapler and the aligning apparatus.
Articles 100a and 100b are designed to be positioned within the receiving area 40 so as to be generally in line with the first and second guide channels 24, 26, respectively. The guide channels 24, 26 will automatically direct the apposed jaws of a surgical stapler into contact with the adhesive surface 105 of each buttress material 100a and 100b when the surgical stapler is clamped down onto the apparatus 10.

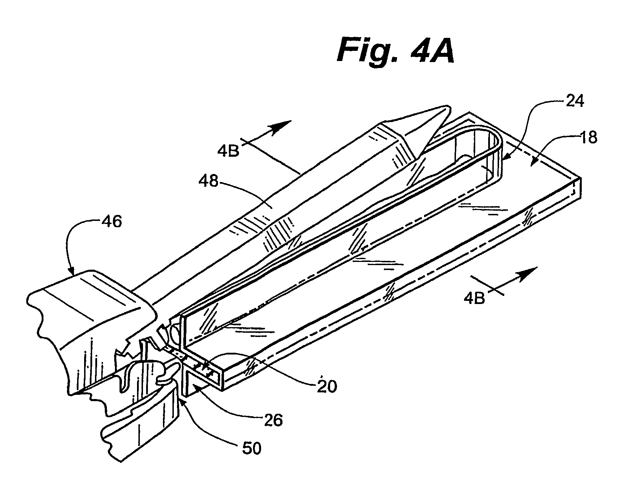

With reference now to Figures 4a and 4b, the apparatus 10 is shown fully enabled for use with a surgical stapler 46. In this embodiment, a combination including articles of buttress materials the articles 100a and 100b and an apparatus 10 for equipping a surgical fastener with a buttress material are provided. The apparatus 10 of this embodiment includes an alignment frame 18, a first article of buttress material 100a and a second article of buttress material 100b. The articles 100a and 100b are releasably disposed within the receiving area 40 so as to be generally aligned with the first and second guide channels 24, 26, respectively.

In order to equip a surgical stapler to produce reinforced staple line, the apposed working surfaces of the surgical stapler are positioned within the first and second guide channels 24, 26, respectively, and clamped down into contact with the adhesive layers 105 of the articles 100a and 100b. The surgical stapler is maintained in this closed position for a given period of time and then pulled away from the alignment frame 18 to remove the pressure equalization means 20, along with the articles of buttress material 100a and 100b from within the receiving area 40. The adhesive layer 105 forms a sufficient bond between articles 100a and 100b and the apposed working surfaces of the surgical stapler such that, when the surgical stapler is opened from the previously clamped position, the articles 100a and 100b remain releasably attached to the apposed working surfaces of the surgical stapler and the pressure equalization member 20 may be removed from between the articles 100a and 100b. The surgical stapler is then fully equipped with articles 100a and 100b such that the apposed working surfaces thereof may be positioned about a section of body tissue to form a reinforced staple line.

While a preferred embodiment of the present invention has been described, it should be understood that various changes, adaptations and modifications may be made therein without departing from the scope of the appended claims.

## Claims

1. A surgical fastener buttress material (100) having an adhesive surface (105) comprising a pre-applied adhesive material, the material (100) being packaged and provided in a manner that permits the adhesive surface (105) to be aligned with and releasably cover a working surface of a surgical fastener (46),
**characterized in that**
the adhesive surface (105) has a predetermined adhesive strength when retained upon the working surface of a surgical fastener (46), the adhesive strength being reduced when the adhesive surface (105) is positioned and hydrated *in vivo.*

2. The material according to claim 1 wherein the buttress material (100) is packaged and provided in a manner that permits the adhesive surface (105) to be aligned with and releasably cover a working surface of a surgical fastener (46) in a one step loading process.

3. The material according to claim 1 wherein the adhesive surface (105) has an adhesive strength sufficient to permit the material, without extraneous means, to be retained upon the working surface of the surgical fastener (46) in the course of the surgical fastener (46) being positioned within the surgical site, and to then be removed from the working surface upon application of the surgical fastener (46).

4. The material according to claim 1 wherein the pre-applied adhesive material comprises a pre-applied hydrogel.

5. The material according to claim 4 wherein the pre-applied adhesive material further includes one or more bioactive agents.

6. The material according to claim 1 wherein the buttress material (100) includes at least one reinforcing material (108) having sufficient mechanical strength to reinforce a surgical fastener suture line.

7. The material according to claim 6 wherein the buttress material (100) includes a composite of two or more materials, with at least one of the materials being a reinforcing material.

8. The material according to claim 6 wherein the at least one reinforcing material (108) substantially resists degradation when implanted *in vivo.*

9. The material according to claim 6 wherein the at least one reinforcing material (108) has a pre-implant tensile strength having a peak load of at least about 2000 grams.

10. The material according to claim 6 wherein the at least one reinforcing material (108) has a pre-implant tensile strength which does not significantly decrease over time when implanted *in vivo.*

11. The material according to claim 6 wherein the at least one reinforcing material (108) has a leak pressure or burst pressure of at least about 90 mm Hg.

12. The material according to claim 6 wherein the at least one reinforcing material (108) comprises a natural animal tissue.

13. The material according to claim 12 wherein the natural animal tissue comprises a fibro-serous or serous membrane.

14. The material according to claim 13 wherein the natural animal tissue comprises a fibro-serous membrane comprising bovine pericardium.

15. The material according to claim 6 wherein the at least one reinforcing material (108) also has an adhesive strength sufficient to permit the material, without extraneous means, to be retained upon the working surface of the surgical fastener (46) in the course of the surgical fastener (46) being positioned within the surgical site, and to then be removed from the working surface upon application of the surgical fastener (46).

16. A combination comprising a surgical fastener (46) having at least one working surface and a buttress material (100) according to claim 1 positioned on the at least one working surface.

17. The combination of claim 16 wherein the surgical fastener comprises a surgical stapler (46).

18. An apparatus (10) for facilitating alignment and application of a buttress material (100) to a working surface of a surgical fastener (46), the device incorporating a buttress material (100) according to claim 1.

19. The apparatus (10) according to claim 18 further comprising an aligning frame (18) which retains one or more articles of the buttress material (100) in releasable attachment with the aligning frame (18) such that the one or more articles will be automatically lined up with apposed working surfaces of a surgical fastener (46).

20. A surgical kit for facilitating application of an article of buttress material (100) to the apposed working surfaces of a surgical fastener (46), the kit including one or more articles of buttress material (100) according to claim 1 and an aligning apparatus (10).

21. The surgical kit according to claim 20 wherein the one or more articles of buttress material (100) are adapted to be loaded into the aligning apparatus (10).

## Patentansprüche

1. Versteifungsmaterial (100) für chirurgische Befestigungsvorrichtungen, welches eine adhäsive Oberfläche (105) aufweist, welche ein vorher aufgetragenes adhäsives Material umfasst, wobei das Material (100) verpackt und auf eine Weise bereitgestellt ist, welche es erlaubt, die adhäsive Oberfläche (105) an einer Arbeitsoberfläche einer chirurgischen Befestigungsvorrichtung (46) auszurichten und diese lösbar abzudecken, **dadurch gekennzeichnet, dass** die adhäsive Oberfläche (105) eine vorbestimmte adhäsive Festigkeit aufweist, wenn sie auf der Arbeitsoberfläche einer chirurgischen Befestigungsvorrichtung (46) gehalten ist, wobei die adhäsive Festigkeit reduziert wird, wenn die adhäsive Oberfläche (105) *in vivo* positioniert und hydratisiert ist.

2. Material nach Anspruch 1, wobei das Versteifungsmaterial (100) verpackt und auf eine Weise bereitgestellt ist, welche es erlaubt, die adhäsive Oberfläche (105) in einem einschrittigen Ladevorgang mit einer Arbeitsoberfläche einer chirurgischen Befestigungsvorrichtung (46) auszurichten und diese lösbar abzudecken.

3. Material nach Anspruch 1, wobei die adhäsive Oberfläche (105) eine adhäsive Festigkeit aufweist, welche ausreichend ist, es dem Material zu erlauben, ohne äußere Mittel, im Zuge der Positionierung der chirurgischen Befestigungsvorrichtung (46) innerhalb der Behandlungsstelle, auf der Arbeitsoberfläche der chirurgischen Befestigungsvorrichtung (46) gehalten zu werden, und dann nach Anbringen der chirurgischen Befestigungsvorrichtung (46) von der Arbeitsoberfläche entfernt zu werden.

4. Material nach Anspruch 1, wobei das voraufgetragene adhäsive Material ein voraufgetragenes Hydrogel umfasst.

5. Material nach Anspruch 4, wobei das voraufgetragene adhäsive Material zusätzlich einen oder mehrere bioaktive Wirkstoffe einschließt.

6. Material nach Anspruch 1, wobei das Versteifungsmaterial (100) zumindest ein Verstärkungsmaterial (108) enthält, welches eine ausreichende mechanische Festigkeit besitzt, um eine Nahtlinie einer chirurgischen Befestigungsvorrichtung zu verstärken.

7. Material nach Anspruch 6, wobei das Versteifungsmaterial (100) einen Verbund aus einem oder mehreren Materialien enthält, wobei zumindest eines dieser Materialien ein Verstärkungsmaterial ist.

8. Material nach Anspruch 6, wobei das zumindest eine Verstärkungsmaterial (108) im Wesentlichen einer Degradierung Stand hält, wenn es *in vivo* implantiert ist.

9. Material nach Anspruch 6, wobei das zumindest eine Verstärkungsmaterial (108) eine präimplantologische Zugfestigkeit besitzt, welche eine Spitzenlast von mindestens rund 2000g hat.

10. Material nach Anspruch 6, wobei das zumindest eine Verstärkungsmaterial (108) eine präimplantologische Zugfestigkeit besitzt, welche im Laufe der Zeit nicht signifikant abnimmt, wenn *in vivo* implantiert.

11. Material nach Anspruch 6, wobei das zumindest eine Verstärkungsmaterial (108) einen Durchlässigkeitsdruck oder Berstdruck von mindestens rund 90mm Hg besitzt.

12. Material nach Anspruch 6, wobei das zumindest eine Verstärkungsmaterial (108) ein natürliches Tiergewebe umfasst.

13. Material nach Anspruch 12, wobei das natürliche Tiergewebe eine fibroseröse oder seröse Membran umfasst.

14. Material nach Anspruch 13, wobei das natürliche Tiergewebe eine fibroseröse Membran umfasst, welche Bovin-Perikat umfasst.

15. Material nach Anspruch 6, wobei das zumindest eine Verstärkungsmaterial (108) ferner eine adhäsive Festigkeit aufweist, welche ausreichend ist, es dem Material zu erlauben, ohne äußere Mittel, im Zuge der Positionierung der chirurgischen Befestigungsvorrichtung (46) innerhalb der Behandlungsstelle, auf der Arbeitsoberfläche des chirurgischen Befestigungsvorrichtung (46) gehalten zu werden, und um dann nach Anbringen der chirurgischen Befestigungsvorrichtung (46) von der Arbeitsoberfläche entfernt zu werden.

16. Kombination, welche eine chirurgische Befestigungsvorrichtung (46) mit zumindest einer Arbeitsoberfläche, und ein Versteifungsmaterial (100) nach Anspruch 1 umfasst, welches auf der zumindest einen Arbeitsoberfläche positioniert ist.

17. Kombination nach Anspruch 16, wobei die chirurgische Befestigungsvorrichtung eine chirurgische Heftvorrichtung (46) umfasst.

18. Vorrichtung (10), welche das Ausrichten und Anbringen eines Versteifungsmaterials (100) auf einer Arbeitsoberfläche einer chirurgischen Befestigungsvorrichtung (46) ermöglicht, wobei das Gerät ein Versteifungsmaterial (100) gemäß Anspruch 1 umfasst.

19. Vorrichtung (10) nach Anspruch 18, weiter umfassend einen Ausrichtrahmen (18), welcher einen oder mehrere Abschnitte des Versteifungsmaterials (100) in lösbarer Verbindung mit dem Ausrichtrahmen (18) hält, so dass der eine Abschnitt oder mehrere Abschnitte automatisch mit gegenüberliegenden Arbeitsoberflächen einer chirurgischen Befestigungsvorrichtung (46) ausgerichtet werden.

20. Chirurgiekit, welches das Anbringen eines Abschnitts des Versteifungsmaterials (100) auf die gegenüberliegenden Arbeitsoberflächen einer chirurgischen Befestigungsvorrichtung (46) ermöglicht, wobei das Kit einen Abschnitt oder mehrere Abschnitte des Versteifungsmaterials (100) gemäß Anspruch 1 und eine Ausrichtvorrichtung (10) beinhaltet.

21. Chirurgiekit nach Anspruch 20, wobei der eine Abschnitt oder die mehreren Abschnitte des Versteifungsmaterials (100) dazu angepasst sind, in die Ausrichtvorrichtung (10) geladen zu werden.

## Revendications

1. Matériau contrefort de moyen de fixation chirurgical (100) présentant une surface adhésive (105) comprenant un matériau adhésif pré-appliqué, le matériau (100) étant emballé et fourni de manière à ce que la surface adhésive (105) puisse être alignée avec et puisse couvrir de façon détachable une surface de travail d'un moyen de fixation chirurgical (46), **caractérisé en ce que** la surface adhésive (105) présente une force adhésive prédéfinie lorsqu'elle est retenue sur la surface de travail d'un moyen de fixation chirurgical (46), la force adhésive étant réduite lorsque la surface adhésive (105) est positionnée et hydratée *in vivo.*

2. Matériau selon la revendication 1, étant donné que le matériau contrefort (100) est emballé et fourni de manière à ce que la surface adhésive (105) puisse être alignée avec et puisse couvrir de façon détachable une surface de travail d'un moyen de fixation chirurgical (46) dans un processus de chargement à une étape.

3. Matériau selon la revendication 1, étant donné que la surface adhésive (105) présente une force adhésive suffisante pour permettre que le matériau, sans moyens externes, soit retenu sur la surface de travail du moyen de fixation chirurgical (46) pendant que le moyen de fixation chirurgical (46) est positionné au sein du site chirurgical, puis soit enlevé de la surface de travail à l'application du moyen de fixation chirurgical (46).

4. Matériau selon la revendication 1, étant donné que le matériau adhésif pré-appliqué comprend un hydrogel pré-appliqué.

5. Matériau selon la revendication 4, étant donné que le matériau adhésif pré-appliqué comporte en outre un ou plusieurs agents bioactifs.

6. Matériau selon la revendication 1, étant donné que le matériau contrefort (100) comporte au moins un matériau de renforcement (108) présentant une résistance mécanique suffisante pour renforcer une ligne de suture de moyen de fixation chirurgical.

7. Matériau selon la revendication 6, étant donné que le matériau contrefort (100) comporte un composite d'au moins deux matériaux, au moins l'un des matériaux étant un matériau de renforcement.

8. Matériau selon la revendication 6, étant donné que le au moins un matériau de renforcement (108) résiste sensiblement à la dégradation lorsqu'il est implanté *in vivo.*

9. Matériau selon la revendication 6, étant donné que le au moins un matériau de renforcement (108) présente une résistance à la traction de pré-implant avec une charge maximale d'au moins environ 2000 grammes.

10. Matériau selon la revendication 6, étant donné que le au moins un matériau de renforcement (108) présente une résistance à la traction de pré-implant qui ne diminue pas considérablement au fil du temps lorsqu'il est implanté *in vivo.*

11. Matériau selon la revendication 6, étant donné que le au moins un matériau de renforcement (108) présente une pression de fuite ou pression d'éclatement d'au moins environ 90 mm Hg.

12. Matériau selon la revendication 6, étant donné que le au moins un matériau de renforcement (108) comprend un tissu animal naturel.

13. Matériau selon la revendication 12, étant donné que le tissu animal naturel comprend une membrane fibroséreuse ou séreuse.

14. Matériau selon la revendication 13, étant donné que le tissu animal naturel comprend une membrane fibroséreuse comprenant du péricarde bovin.

15. Matériau selon la revendication 6, étant donné que le au moins un matériau de renforcement (108) présente également une force adhésive suffisante pour permettre que le matériau, sans moyens externe, soit retenu sur la surface de travail du moyen de fixation chirurgical (46) pendant que le moyen de fixation chirurgical (46) est positionné au sein du site chirurgical, puis soit enlevé de la surface de travail à l'application du moyen de fixation chirurgical (46).

16. Combinaison comprenant un moyen de fixation chirurgical (46) présentant au moins une surface de travail et un matériau contrefort (100) selon la revendication 1 positionné sur la au moins une surface de travail.

17. Combinaison selon la revendication 16, dans laquelle le moyen de fixation chirurgical comprend une agrafeuse chirurgicale (46).

18. Dispositif (10) destiné à faciliter l'alignement et l'application d'un matériau contrefort (100) avec/à une surface de travail d'un moyen de fixation chirurgical (46), le dispositif incorporant un matériau contrefort (100) selon la revendication 1.

19. Dispositif (10) selon la revendication 18, comprenant en outre un cadre d'alignement (18) qui retient au moins un article en ledit matériau de contrefort (100) attaché de manière détachable au cadre d'alignement (18) de manière à ce que les au moins un articles soient automatiquement alignés avec les surfaces de travail apposées d'un moyen de fixation chirurgical (46).

20. Kit chirurgical destiné à faciliter l'application d'un article en un matériau contrefort (100) sur les surfaces de travail apposées d'un moyen de fixation chirurgical (46), le kit comportant au moins un article en matériau contrefort (100) selon la revendication 1 et un dispositif d'alignement (10).

21. Kit chirurgical selon la revendication 20, dans lequel les au moins un articles en matériau contrefort (100) sont adaptés de manière à être chargés dans le dispositif d'alignement (10).
